# EUROPEAN PATENT APPLICATION

(11) **EP 3 881 893 A1**
(43) Date of publication of application: **22.09.2021**
(21) Application number: 20163493.8
(22) Date of filing: 17.03.2020
(51) Int. Cl.: A61N 1/375, A61N 1/39, A61N 1/36

(54) **IMPLANTABLE PULSE GENERATOR COMPRISING AN ARRANGEMENT OF TWO COMPONENTS ARRANGED AT AN ANGLE TO EACH OTHER**

(71) Applicant: BIOTRONIK SE & Co. KG, 12359 Berlin (DE)
(72) Inventor: DÖRR, Thomas, 12437 Berlin (DE); WEISS, Ingo, 12435 Berlin (DE)
(74) Representative: Biotronik Corporate Services SE

(57) **Abstract**

The present invention relates to an implantable pulse generator (1), comprising a housing (200) surrounding an internal space (200a) of the housing (1), and at least a first and a second component (101, 102) of the pulse generator (1), wherein the first and the second component (101, 102) are arranged in the internal space (200a), wherein the first component (101) comprises a height being smaller than an extension of the first component (101) in a width direction and in a depth direction of the first component (101), and wherein the second component (102) comprises a height being smaller than an extension of the second component (102) in a width direction and in a depth direction of the second component (102), wherein each of the first and the second component (101, 102) comprises an outer surface (101a, 102a) extending along the respective width and depth direction. According to the present invention, said outer surfaces (101a, 102a) of the first and the second component (101, 102) are each flat, wherein a first alignment plane (103) aligned with the outer surface (101a) of the first component (101) is arranged at a first angle (105) to a second alignment plane (104) aligned with the outer surface (102a) of the second component (102), wherein the first angle (105) is larger than zero and smaller than 90°.

## Description

The present invention relates to an implantable pulse generator. Such pulse generators can be applied in different therapies that require the generation of electrical stimulation pulses.

Possible applications (among others) are implantable cardiac pacemakers, implantable cardioverter-defibrillators, but also neurostimulators.

Usually, implantable pulse generators are designed to have at least one flat base surface, wherein frequently mounting frames are used that spatially align the components in such a way that at least one base surface of the respective component is coplanar with a base surface of at least one other component and parallel to one of the housing surfaces.

US7103415B2 discloses a housing shape with a non-planar footprint.

Furthermore, EP1870130A1 describes a housing of an implantable electrostimulator comprising two complementary coupled half-shells that each comprise a curved shape and together enclose correspondingly curved components of the electrostimulator.

However, implants with flat base surfaces do not follow the curved body shapes (especially rib curvature). At the same time, they cannot be implanted in the best possible space-saving manner and may cause pressure points at their edges. They are also cosmetically disadvantageous.
Furthermore, curved housing shells and correspondingly curved components have the disadvantage that bespoke components are needed and ordinary components cannot be used. Based on the above, the problem to be solved by the present invention therefore is to provide an implantable pulse generator that can be produced in a comparatively easy and cost-effective manner but still allows to adapting the shape of the pulse generator to the anatomy of the patient to avoid the above-mentioned disadvantages relating to planar footprints.

This problem is solved by an implantable pulse generator having the features of claim 1.

Preferred embodiments of this aspect of the present invention are stated in the sub claims and are described below.

According to claim 1 an implantable pulse generator is disclosed, comprising:
- a housing surrounding an internal space of the housing, and
- at least a first and a second component of the pulse generator, wherein the first and the second component are arranged in the internal space, wherein the first component comprises a height being smaller than an extension of the first component in a width direction and in a depth direction of the first component, and wherein the second component comprises a height being smaller than an extension of the second component in a width direction and in a depth direction of the second component, and wherein the first component comprises an outer surface extending along the width direction and the depth direction of the first component, and wherein the second component comprises an outer surface extending along the width direction and the depth direction of the second component.

In the sense of the present invention, electrical stimulation is understood as an electrical pulse for pacing heart tissue, or a shock stimulation pulse for achieving electrical defibrillation, or any other pulse form for stimulation of the heart tissue.

According to the present invention, said outer surfaces are flat, wherein a first alignment plane running parallel to the outer surface of the first component encloses a first angle with a second alignment plane running parallel to the outer surface of the second component, wherein the first angle is larger than zero and smaller than 90°.

In other words, the invention is based on the idea to arrange components of an implantable pulse generator in a non-planar, i.e. especially angled, arrangement so that the housing of the implant can be made to fit the body's anatomy at the implantation site.

According to a preferred embodiment, said first angle is smaller than 80°, particularly smaller than 70°, particularly smaller than 60°, particularly smaller than 50°, particularly smaller than 45°.

Furthermore, the first and the second component each form an electronic component of the implantable pulse generator. Furthermore, according to an embodiment, the first and the second component are electrically connected to one another.

According to a further embodiment, the housing of the implantable pulse generator comprises an outer surface that is preferably configured to face at least one rib of a patient in an implanted state of the implantable pulse generator, wherein said outer surface comprises a flat first section and an adjacent flat second section which are arranged at a non-zero second angle, wherein particularly the first component extends along the first section, and wherein particularly the second component extends along the second section, and wherein the two sections of said outer surface of the housing are arranged at a third angle of less than 45° to at least one of the alignment planes.

Particularly, in the sense of the present invention, a component is denoted a "flat component" in case its height or extension in the height direction is smaller than its extension along any other two orthogonal axes, e.g. along the width and depth axes or directions that are orthogonal to the height direction. More specifically, according to an embodiment, the respective component is considered to be a flat component, in case the extension in the height direction is smaller than both other extensions multiplied by a factor of 0.7 (for example in case the first component comprises a width and a depth of 10mm, respectively, the height is smaller than 0.7^{∗}10mm=7mm). Preferably said factor is 0.6, particularly 0.5, particularly 0.4, particularly 0.3, particularly 0.2, particularly 0.1.

Furthermore, generally, the respective alignment plane of a flat component can be defined by the mass points of an isovolumetric discretization (e.g. voxel approximation) of the component surface obtained after minimization of an error function in the sense of one of the L1, L2,...,L-infinity standards (e.g. by the method of least squares). In particular, the alignment plane can be defined by generating a best approximation to a cloud of points of the component surface. The best approximation is for instance found by minimizing the error function in the sense of the method of least squares.

Particularly, the first and the second component can each be a discrete electronic component, or an integrated circuit, or a module comprised of several electrically connected parts.

Preferably, according to an embodiment, the first and the second component comprise a size such that they occupy together at least 5% of the inner volume of the housing of the implantable pulse generator.

Particularly, in an embodiment, the first component is one of: an energy storing device, a battery, a capacitor, a transformer. Likewise, the second component can be one of: an energy storing device, a battery, a capacitor, a voltage transformer.

Furthermore, according to a preferred embodiment, the implantable pulse generator is configured to be implanted in the area of the thorax of the patient adjacent at least one rib of the patient. For instance, the smallest distance between a rip and a point of the housing of the implantable pulse generator is 10 cm or less.

Preferably, said second angle at which the two sections are arranged is selected such that the outer side of the housing is capable of following a curvature of the at least one rib of the patient in the implanted state of the implantable pulse generator, and/or wherein said first angle at which the alignment planes are arranged is selected such that the components follow a curvature of the at least one rib of the patient.

According to yet another embodiment, the implantable pulse generator is configured to generate electrical stimulation pulses for anti-bradycardia or anti-tachycardia pacing of the heart of the patient.

Alternatively, according to a further embodiment, the implantable pulse generator is configured to perform neurostimulation.

According to a further embodiment, the implantable pulse generator is an implantable cardioverter-defibrillator (ICD), wherein the first component is an energy storage device, particularly a battery or at least one high-voltage capacitor, particularly for supplying an ICD function of the pulse generator with electrical energy, e.g. for delivering an electrical shock to the patient, and wherein the second component is an energy storage device, particularly a battery or at least one high-voltage capacitor, particularly for supplying an ICD function of the pulse generator with electrical energy, e.g. for delivering an electrical shock to the patient. Particularly, both the first and the second component can be a battery. Furthermore, both the first and the second component can be a high-voltage capacitor. Further, one of the first and the second component can be a battery while the other component can be a high-voltage capacitor.

According to a further embodiment, the pulse generator comprises a mounting frame that holds the first and the second component such that said alignment planes are arranged at said first angle. This is advantageous, since the two components can be brought in the correct spatial position with respect to one another and can be electrically connected to one another before being arranged in the internal space of the housing using the mounting frame which simplifies the assembly.

According to a further embodiment, the mounting frame comprises at least a first fastening element for the first component, wherein the first fastening element is configured to engage with the first component to form a snap-in-connection when the first component is arranged in the mounting frame so as to fasten the first component with respect to the mounting frame. Furthermore, according to an embodiment, the mounting frame comprises at least a second fastening element for the second component, wherein the second fastening element is configured to engage with the second component to form a snap-in-connection when the second component is arranged in the mounting frame so as to fasten the second component with respect to the mounting frame.

According to a further embodiment, the housing of the implantable pulse generator comprises an inside facing the internal space of the housing, wherein the inside comprises a first section and a second section, wherein the first and the second section of the inside are arranged at an angle such that the first and the second alignment plane are arranged at the first angle when the first component rests on the first section of the inside and the second component rests on the second section of the inside of the housing. Particularly, the first section and the second section of the inside can correspond to locally flat surfaces of the inside of the housing.

Furthermore, according to an embodiment, the first and the second component are connected to the housing by means of an adhesive, so as to prevent slippage of the components in the internal space of the housing.

According to yet another embodiment, the first and the second component each comprise an outer lateral surface, wherein the two lateral surfaces face each other and extend parallel to one another. Alignment of the two lateral surfaces allows to arranging the two adjacent components with a minimal gap in between them despite their angled configuration so that installation space can be advantageously reduced.

Furthermore, according to yet another embodiment, the volume of the implantable pulse generator's housing is preferably smaller than 70cm³.

In the following embodiments, further features and advantages of the present invention shall be described with reference to the Figures which depict preferred embodiments of the present invention, wherein
- Fig. 1: shows a schematical illustration of two components of an implantable pulse generator that are arranged at an angle;
- Fig. 2: shows a schematical illustration of two components of an implantable pulse generator that are arranged at an angle in an internal space of a housing of the implantable pulse generator;
- Fig. 3: shows a schematical illustration of determining an alignment plane based on a voxel approximation of a surface of the component in question;
- Fig. 4: shows a mounting frame for holding two components at an angle; and
- Fig. 5: shows a schematical illustration of two components of an implantable pulse generator having aligned lateral surfaces.

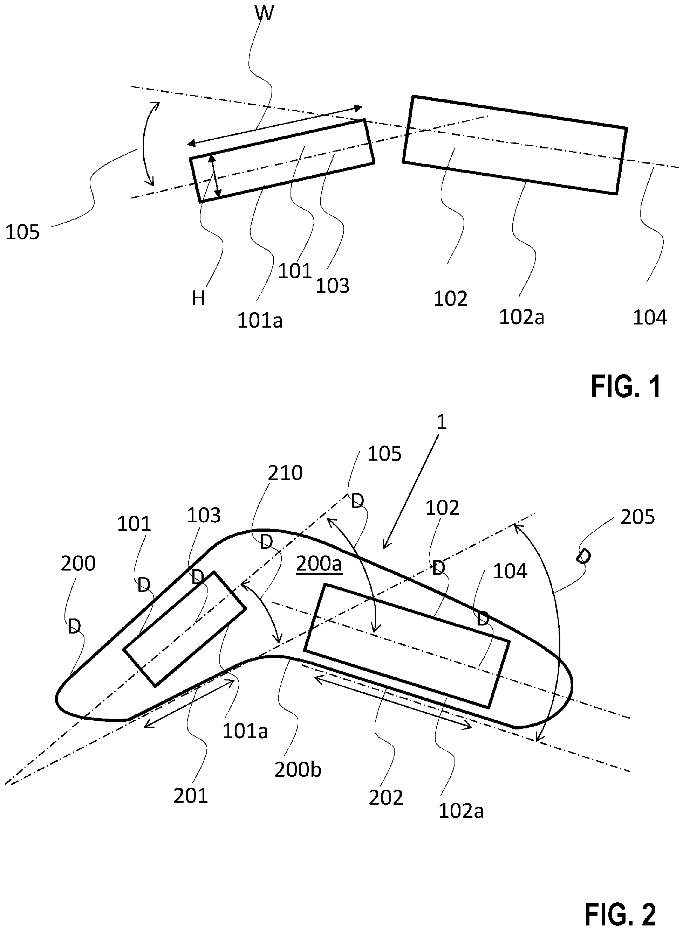

The present invention relates to an implantable pulse generator 1 comprising a housing 200 (cf. e.g. Fig. 2) surrounding an internal space 200a of the housing 200, and at least a flat first and a flat second component 101, 102 of the pulse generator 1, which components 101, 102 are arranged at a non-vanishing angle as indicated in Figs. 1 and 2. The height direction H and the width direction W of the first component 101 are indicated in Fig. 1 for example, wherein the depth direction of the first component runs orthogonal to the height and width direction H, W. These directions are analogously defined for the second component 102.

The fact that the components 101, 102 are flat essentially means that each of the two components 101, 102 comprises a height being smaller than an extension of the respective component in the width direction and in the depth direction of the respective component 101, 102 (see also above). In this regard each of the first and the second component 101, 102 comprises a flat outer surface 101a, 102a extending along the width direction and the depth direction, which outer surfaces face towards the patient anatomy that shall be matched. According to the present invention (cf. e.g. Fig. 2), a first alignment plane 103 aligned with the flat outer surface 101a of the first component 101 is arranged at a first angle 105 to a second alignment plane 104 of the flat outer surface 102a of the second component 102, wherein the first angle 105 is preferably larger than zero and smaller than 90°.

Furthermore, said housing 200 comprises an inwardly outer surface 200b comprising at least two flat sections 201, 202 which are arranged at a non-zero second angle 205 with respect to one another and which are arranged at a third angle 210 of less than 45 degrees with respect to least one of the alignment planes 103, 104 of the inner components 101, 102. This third angle 210 preferably amounts to 0° degrees. The outer surface 200b of the housing 200 is configured to face the thorax of the person and advantageously allows adapting the housing 200 of the pulse generator 1 to a curvature of the rib(s) of the person.

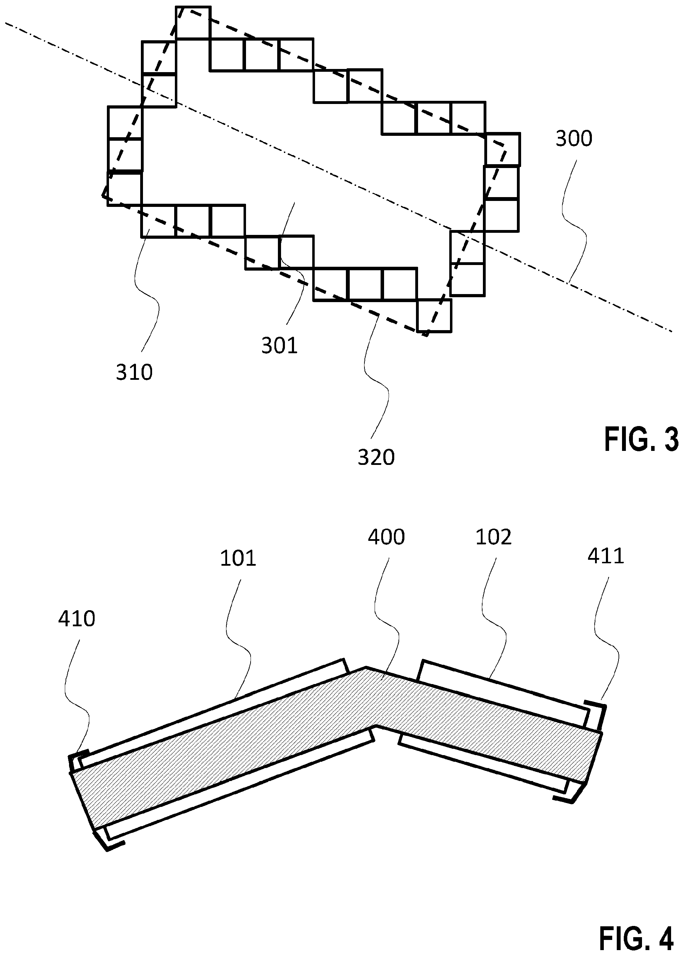

Fig 3 shows an alignment plane 300 of a flat component 301 according to the invention. Generally, the alignment plane 300 can be defined as a compensation plane by the mass points 310 of an isovolumetric discretization (e.g. a voxel approximation) of the component's outer surface 320 which is obtained after minimizing an error function in the sense of one of the L1, L2, ..., L-infinity standards (usually by the method of least squares).
For instance, the cloud of points is defined by the mass points of squares 310, whereby plane 300 is the approximation of said mass points with a minimum error in the sense if the method of least squares.

According to an embodiment depicted schematically in Fig. 4, the implantable pulse generator 1 can comprise a mounting frame 400 that is configured to hold the components 101, 102 such that they assume said angled arrangement with respect to one another once they are held by the mounting frame. The components 101, 102 are optionally fastened to the mounting frame 400 by means of fastening elements 410, 411, wherein each fastening element 410, 411 engages with a portion of an associated component 101, 102 to form a snap-in-connection that fixes the respective component 101, 102 with respect to the mounting frame 400 which then extends around both components 101, 102, in particular.

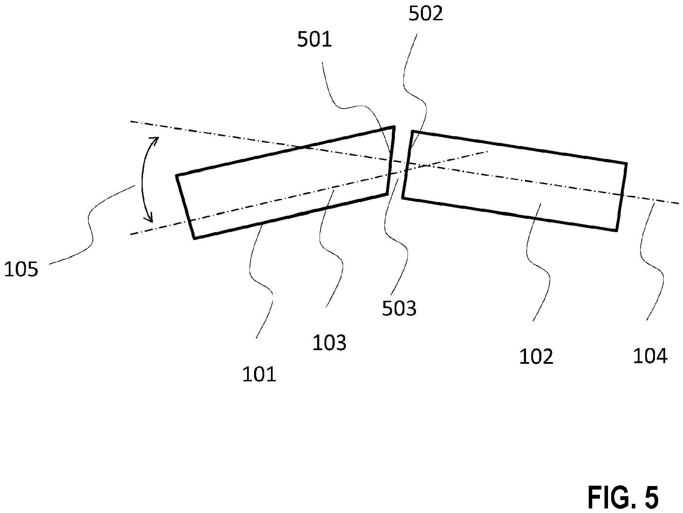

Furthermore, apart form said outer surfaces 101a, 102a of the two components 101, 102 that assume an angled configuration, also lateral surfaces 501, 502 of the components 101, 102 can be adapted with respect to one another to minimize installation space. Particularly, as shown in Fig. 5 the two components 101, 102 each comprise a lateral surface 501, 502, wherein said lateral surfaces 501, 502 face each other and extend parallel to one another so that a gap 503 between the two lateral surfaces 501, 502 is minimal despite said angled configuration of the two components 101, 102.

The solution according to the present invention allows providing a housing of an implantable pulse generator that comprises a physiologically favorable shape so that it follows the curves of the body. The surfaces of the components can also be arranged in such a way that the gap between adjacent angled components is minimal.

## Claims

1. An implantable pulse generator (1), comprising:
- a housing (200) surrounding an internal space (200a) of the housing (1), and
- at least a first and a second component (101, 102) of the pulse generator (1), wherein the first and the second component (101, 102) are arranged in the internal space (200a), wherein the first component (101) comprises a height being smaller than an extension of the first component (101) in a width direction and in a depth direction of the first component (101), and wherein the second component (102) comprises a height being smaller than an extension of the second component (102) in a width direction and in a depth direction of the second component (102), wherein each of the first and the second component (101, 102) comprises an outer surface (101a, 102a) extending along the respective width and depth direction,
**characterized in that**
said outer surfaces (101a, 102a) of the first and the second component (101, 102) are each flat, wherein a first alignment plane (103) aligned with the outer surface (101a) of the first component (101) is arranged at a first angle (105) to a second alignment plane (104) aligned with the outer surface (102a) of the second component (102), wherein the first angle (105) is larger than zero and smaller than 90°.

2. The implantable pulse generator according to claim 1, **characterized in that** the first and the second component (101, 102) are electrically connected to one another.

3. The implantable pulse generator according to one of the preceding claims, **characterized in that** the housing (200) comprises an inwardly curved outer surface (200b), that is preferably configured to face at least one rib of a patient in an implanted state of the implantable pulse generator (1), wherein said outer surface (200b) of the housing (200) comprises a flat first section (201) and an adjacent flat second section (202) which are arranged at a non-zero second angle (205), wherein the two sections (201, 202) are arranged at a third angle (210) of less than 45° to at least one of the alignment planes (103, 104).

4. The implantable pulse generator according to one of the preceding claims, **characterized in that** the first and the second component (101, 102) comprise a size such that they occupy at least 5% of the inner volume of the housing (200) of the implantable pulse generator (1).

5. The implantable pulse generator according to one of the preceding claims, **characterized in that** the first component (101) is one of: an energy storing device, a battery, a capacitor, a transformer; and/or wherein the second component (102) is one of: an energy storing device, a battery, a capacitor, a voltage transformer.

6. The implantable pulse generator according to claim 3 or according to one of the claims 4 to 5 when referring to claim 3, **characterized in that** the implantable pulse generator (1) is configured to be implanted in the area of the thorax adjacent at least one rib of the patient.

7. The implantable pulse generator according to one of the preceding claims, **characterized in that** said second angle (205) at which the two sections (201, 202) of the outer surface (200b) of the housing (200) are arranged is selected such that the outer surface (200b) of the housing (200) is capable of following a course of the at least one rib of the patient in the implanted state of the implantable pulse generator (1), and/or wherein said first angle (105) at which the alignment planes (103, 104) are arranged is selected such that the components (101, 102) follow a course of the at least one rib of the patient.

8. The implantable pulse generator according to one of the preceding claims, **characterized in that** the implantable pulse generator (1) is configured to generate electrical stimulation pulses for anti-bradycardia or anti-tachycardia pacing of the heart of the patient.

9. The implantable pulse generator according to one of the claims 1 to 7, **characterized in that** the implantable pulse generator is configured to perform neurostimulation.

10. The implantable pulse generator according to one of the claims 1 to 8, **characterized in that** the implantable pulse generator (1) is an implantable cardioverter-defibrillator (ICD), wherein the first component (101) is an energy storage device, particularly a battery or at least one high-voltage capacitor, and wherein the second component (102) is an energy storage device, particularly a battery or at least one high-voltage capacitor.

11. The implantable pulse generator according to one of the preceding claims, **characterized in that** the pulse generator (1) comprises a mounting frame (400) that holds the first and the second component (101, 102) such that said alignment planes (103, 104) are arranged at said first angle (105).

12. The implantable pulse generator according to claim 11, **characterized in that** the mounting frame (400) comprises at least a first fastening element (410) for the first component (101), wherein the first fastening element (410) is configured to engage with the first component (101) to form a snap-in-connection when the first component (101) is arranged in the mounting frame (400) so as to fasten the first component (101) to the mounting frame (400), and/or wherein the mounting frame (400) comprise at least a second fastening element (411) for the second component (102), wherein the second fastening element (411) is configured to engage with the second component (102) to form a snap-in-connection when the second component (102) is arranged in the mounting frame so as to fasten the second component (102) to the mounting frame (400).

13. The implantable pulse generator according to one of the preceding claims, **characterized in that** the housing (200) comprises an inside facing the internal space (200a) of the housing (200), wherein the inside comprises a first section and a second section, wherein the first and the second section of the inside are arranged at an angle such that the first and the second alignment plane (103, 104) are arranged at the first angle (105) when the first component (101) rests on the first section of the inside and the second component (102) rests on the second section of the inside of the housing (200).

14. The implantable pulse generator according to one of the preceding claims, **characterized in that** the first and the second component (101, 102) each comprise an outer lateral surface (501, 502), wherein the two lateral surfaces (501, 502) face each other and extend parallel to one another.

15. The implantable pulse generator according to one of the preceding claims, **characterized in that** the volume of the housing (200) of the pulse generator (1) is smaller than 70cm³.
